# EUROPEAN PATENT APPLICATION

(11) **EP 1 688 505 A2**
(43) Date of publication of application: **09.08.2006**
(21) Application number: 06001564.1
(22) Date of filing: 25.01.2006
(51) Int. Cl.: C12Q 1/68

(54) **Method of hybridizing nucleic acids**

(30) Priority: 02.02.2005 KR 2005009743
(71) Applicant: Samsung Electronics Co., Ltd., Suwon-si, Gyeonggi-Do (KR)
(72) Inventor: Park, Joon-Shik, Giheung-gu, Yongin-si, Gyeonggi-do (KR); Lee, Myo-yong, Suwon-si, Gyeonggi-do (KR)
(74) Representative: Grünecker, Kinkeldey, Stockmair & Schwanhäusser Anwaltssozietät

(57) **Abstract**

Provided are a method of hybridizing genes, including applying to a gene microarray a hybridization solution in which a compound represented by formula (1) is added: 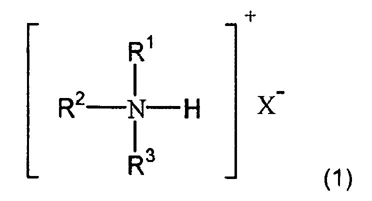 where each of R¹, R², and R³ is independently a straight or branched C₁-C₅ alkyl; and X- is an anion of an organic acid; and a method of using a compound represented by formula (1) as an additive to a hybridization solution.

### Annex to the application documents - subsequently filed sequences listing

<110>	Samsung Electronics Co. Ltd.

<120>	Method of hybridizing gene

<130>	EP38083FZpau

<140>	EP 06 001 564.1
<141>	2006-01-25

<150>	KR 10-2005-0009743
<151>	2005-02-02

<160>	26

<170>	KopatentIn 1.71

<210>	1
<211>	19
<212>	DNA
<213>	Artificial Sequence

<220>
<223>	DNA probe

<400>	1
atgacaatga gtatgccta	19

<210>	2
<211>	19
<212>	DNA
<213>	Artificial Sequence

<220>
<223>	DNA probe

<400>	2
atgacaatca gtatgccta	19

<210>	3
<211>	19
<212>	DNA
<213>	Artificial Sequence

<220>
<223>	DNA probe

<400>	3
taggcatact cattgtcat	19

<210>	4
<211>	25
<212>	DNA
<213>	Artificial Sequence

<220>
<223>	DNA probe

<400>	4
tctaaactga gccagctgca gacgg	25

<210>	5
<211>	25
<212>	DNA
<213>	Artificial Sequence

<220>
<223>	DNA probe

<400>	5
ctggacaagg gggagtcctg cggcg	25

<210>	6
<211>	25
<212>	DNA
<213>	Artificial Sequence

<220>
<223>	DNA probe

<400>	6
gcggcggtcg aggggagctg gctga	25

<210>	7
<211>	25
<212>	DNA
<213>	Artificial Sequence

<220>
<223>	DNA probe

<400>	7
tctgtgcctg cagagttcac ccatg	25

<210>	8
<211>	25
<212>	DNA
<213>	Artificial Sequence

<220>
<223>	DNA probe

<400>	8
aagagcccac aggtgatgag ctacc	25

<210>	9
<211>	25
<212>	DNA
<213>	Artificial Sequence

<220>
<223>	probe

<400>	9
tgttccaggc ctatgagagg cagaa	25

<210>	10
<211>	25
<212>	DNA
<213>	Artificial Sequence

<220>
<223>    probe

<400>    10
gagacgctag tggaggagtg caata                                          25

<210>    11
<211>    25
<212>    DNA
<213>    Artificial Sequence

<220>
<223>    probe

<400>    11
cctgcgctgc ccgctcacag ctccc                                          25

<210>    12
<211>    25
<212>    DNA
<213>    Artificial Sequence

<220>
<223>    probe

<400>    12
ctctccccca gtaaggtcca cggta                                          25

<210>    13
<211>    25
<212>    DNA
<213>    Artificial Sequence

<220>
<223>    probe

<400>    13
gcttccctct ccaggtgtgc gctat                                          25

<210>    14
<211>    25
<212>    DNA
<213>    Artificial Sequence

<220>
<223>    probe

<400>    14
aagccaagct ggtgagtgtc cttgc                                          25

<210>    15
<211>    25
<212>    DNA
<213>    Artificial Sequence

<220>
<223>    probe

<400>    15
gcttcccctc gtaggtctca gcagc                                          25

<210>    16
<211>    25
<212>    DNA
<213>    Artificial Sequence

<220>
<223>    probe

<400>    16
cttggagcag acatccccag gcctc                                          25

<210>    17
<211>    25
<212>    DNA
<213>    Artificial Sequence

<220>
<223>    probe

<400>    17
cacagagtgt gccggtcatc aacag                                          25

<210>    18
<211>    25
<212>    DNA
<213>    Artificial Sequence

<220>
<223>    probe

<400>    18
cacctcagac actgaggcct ccagt                                          25

<210>    19
<211>    25
<212>    DNA
<213>    Artificial Sequence

<220>
<223>    probe

<400>    19
caggaccctg ccggcatcca gcacc                                          25

<210>    20
<211>    25
<212>    DNA
<213>    Artificial Sequence

<220>
<223>    probe

<400>    20
tgtcctccag cagcctggtg ctgta                                          25

<210> 21
<211> 25
<212> DNA
<213> Artificial Sequence

<220>
<223> probe

<400> 21
ccccagctcc aatgtaaaca gaaca 25

<210> 22
<211> 25
<212> DNA
<213> Artificial Sequence

<220>
<223> probe

<400> 22
gggctgaagt ccaaagttca gtccc 25

<210> 23
<211> 25
<212> DNA
<213> Artificial Sequence

<220>
<223> probe

<400> 23
agtggggttt tgggggggca gtggg 25

<210> 24
<211> 25
<212> DNA
<213> Artificial Sequence

<220>
<223> probe

<400> 24
taccgtggac cttactgggg gagag 25

<210> 25
<211> 25
<212> DNA
<213> Artificial Sequence

<220>
<223> probe

<400> 25
tacagcacca ggctgctgga ggaca 25

<210> 26
<211> 25
<212> DNA

<213>    Artificial Sequence

<220>
<223>    probe


<400>    26
tgttctgttt acattggagc tgggg                                              25

## Description

### BACKGROUND OF THE INVENTION

### 1. Field of the Invention

The present invention relates to a method of hybridizing genes in which a hybridization solution is added to a gene microarray, and more particularly, to a method of hybridizing genes for increasing a hybridization specificity to a perfect match probe by increasing a ratio (PM/MM) of a hybridization intensity of the perfect match probe (PM) to a hybridization intensity of a mismatch probe (MM) while increasing or maintaining the PM.

### 2. Description of the Related Art

A gene microarray is a biochip used for the analysis of genes in a sample. The analysis of genes in a sample can be accomplished by adding the sample to a gene probe immobilized on a substrate, and allowing genes in the sample, which has a complementary sequence to the gene probe, to hybridize with the gene probe. In the gene hybridization performed to analyze genes in a sample using such a gene microarray, accurate genetic analysis can be achieved when only a target sample having a base sequence that is completely complementary to the base sequence of a gene probe on a gene microarray hybridizes with the gene probe. However, in reality, a sample having a base sequence in which one or more bases are not complementary to the base sequence of a gene probe on a gene microarray is hybridized with the gene probe (mismatch probe) to achieve a complementary bonding. The hybridization between a probe on a gene chip and a sample DNA having a base sequence mismatched to the probe results in an error in an analysis of genes in a sample. Thus, to accurately analyze genes when a sample hybridizes with a probe on a gene microarray, a hybridization intensity of mismatch probes (MM) must be reduced and a hybridization intensity of perfect match probes (PM) must be increased, thereby increasing a PM/MM ratio.

Conventionally, a method for increasing the PM/MM ratio used in the hybridization of genes has been primarily accomplished by applying a severe condition to the hybridization of genes. Examples of the severe condition include a low NaCl concentration in a hybridization solution as a sample, a high hybridization temperature, the inclusion of a denaturant in a hybridization solution, etc. These severe conditions can increase the PM/MM ratio since the decrease in the hybridization intensity of a perfect match probe caused by the severe conditions is greater than the decrease in the hybridization intensity of a mismatch probe caused by the severe conditions (U.S. Patent No. 6,632,605).

However, while the severe conditions described above can increase the PM/MM ratio, they decrease an absolute hybridization intensity of the perfect match probe, resulting in a reduction in measurement sensitivity.

In particular, the high hybridization temperature may cause genes immobilized on a substrate of a gene microarray to separate from the substrate (U.S. Patent No. 6,632,605).

Primarily used denaturants include organic denaturants such as formamide, formaldehyde, glycerol, DMSO, DMF, GuSCN, and urea. However, such organic denaturants are salted out due to low solubility in the hybridization solution, which makes it difficult to use the denaturants in a high concentration. Furthermore, the organic denaturants are toxic, and thus should not be directly handled by a user.

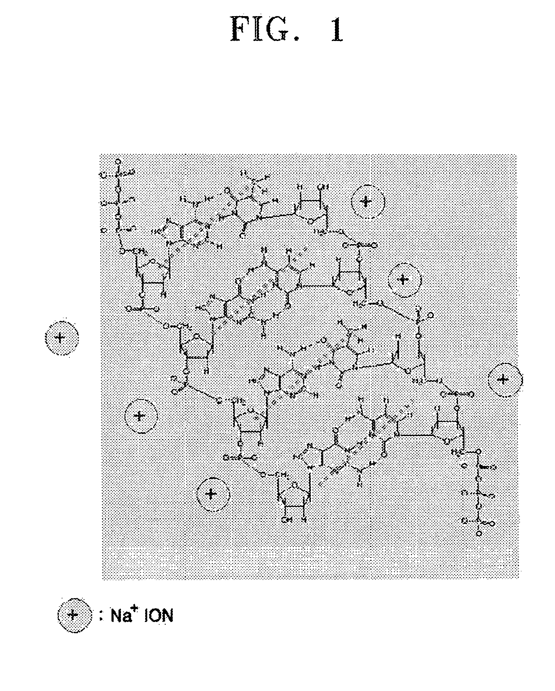

Meanwhile, NaCl is generally used as an additive in a hybridization solution to stabilize hybridized genes in the hybridization of genes. A sodium cation of NaCl ionically binds to an oxygen anion of phosphate from a gene due to electrostatic attraction, thereby reducing instability of hybridization due to repulsion between phosphate anions of nucleic acids to stabilize the hybridization of genes. FIG. 1 illustrates where a sodium cation binds to a phosphate anion due to an electrostatic interaction in a hybridization of genes. The reduction in the concentration of NaCl salt which stabilizes the hybridization of genes is a severe condition for the hybridization of genes as described above, and can increase the PM/MM ratio, but decreases the hybridization intensity of a perfect match probe.

Tetramethyl ammonium chloride (TMAC) has been proposed as an additive in the hybridization of genes on a gene microarray in Proc. Natl. Acad. Sci. USA, 198582: 1585-1588. In addition, U.S. Patent No. 6, 632,605 discloses the use of betaine, which is a quaternary ammonium salt, as an additive in the hybridization of genes on a microarray. However, TMAC and betaine do not increase the PM/MM ratio, but allow a difference in hybridization intensities to depend on only the length of a base sequence of a nucleic acid probe and not on the type of base sequence by making a hydrogen bond intensity between adenine and thymine of a base sequence of a nucleic acid probe similar to a hydrogen bond intensity between cytosine and guanine.

Thus, there is demand for a novel hybridization method capable of increasing the PM/MM ratio while increasing or maintaining the intensity of a perfect match probe.

### SUMMARY OF THE INVENTION

The present invention provides a method of hybridizing genes capable of increasing a PM/MM ratio while increasing or maintaining the hybridization intensity of a perfect match probe when hybridization is performed on a gene microarray.

The present invention also provides an additive capable of increasing the PM/MM ratio while increasing or maintaining the hybridization intensity of a perfect match probe by being added to a hybridization solution as a sample to be applied to a gene microarray.

According to an aspect of the present invention, there is provided a method of hybridizing genes, including applying to a gene microarray a hybridization solution in which a compound represented by formula (1) is added: 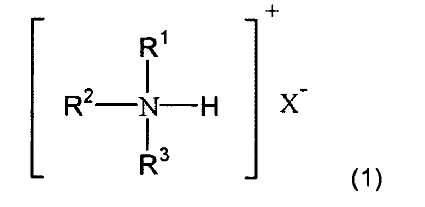 where each of R¹, R², and R³ is independently a straight or branched C₁-C₅ alkyl; and X- is an anion of an organic acid.

In formula (1), each of R¹, R², and R³ may be independently ethyl, n-propyl, or iso-propyl, but is not limited thereto.

In formula (1), X- may be formate, acetate, propionate, butyrate, p-toluene sulfonate, benzene sulfonate, trifluoroacetate, oxalate, or tartrate, but is not limited thereto.

The compound represented by formula (1) may be triethylammonium acetate or N,N-diisopropylethylammonium acetate.

According to another aspect of the present invention, there is provided an additive comprising a compound represented by formula (1) for addition to a hybridization solution applied to a gene microarray: 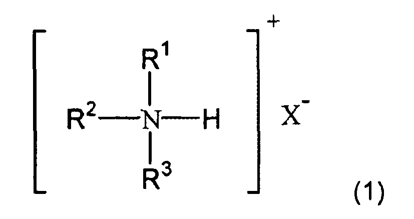 where each of R¹, R², and R³ is independently a straight or branched C₁-C₅ alkyl; and X- is an anion of an organic acid.

In formula (1), each of R¹, R², and R³ may be independently ethyl, n-propyl, or iso-propyl.

In formula (1), X- may be formate, acetate, propionate, butyrate, p-toluene sulfonate, benzene sulfonate, trifluoroacetate, oxalate, or tartrate.

The compound represented by formula (1) may be triethylammonium acetate or N, N-diisopropylethylammonium acetate.

### BRIEF DESCRIPTION OF THE DRAWINGS

The above and other features and advantages of the present invention will become more apparent by describing in detail exemplary embodiments thereof with reference to the attached drawings in which:
FIG. 1 illustrates a structure in which a sodium cation is bound to a phosphate anion of a hybridized ds-DNA due to electrostatic interaction;
FIG. 2 illustrates a structure in which a trialkylammonium ion derived from a compound represented by formula (1) is bound to a phosphate anion of a hybridized ds-DNA due to electrostatic interaction;
FIG. 3 illustrates in detail the structure in which triethylammonium acetate, one of the compound represented by formula (1) is bound to a phosphate anion of a hybridized ds-DNA due to electrostatic interaction;
FIG. 4 illustrates the spot arrangement of genes when manufacturing a gene microarray in Example 1;
FIG. 5 illustrates a patch attached to a microarray for hybridization and the introduction of a hybridization solution to the microarray through the patch;
FIG. 6A illustrates the scanning results of hybridized microarrays for the control and the treatment with 0.75 M TEAA using an Axon Scaner;
FIG. 6B illustrates the results of scanning hybridized microarrays respectively treated with 0.5 M, 0.75 M, and 1.0 M TEAA using an Axon Scaner;
FIG. 6C illustrates the results of scanning hybridized microarrays respectively treated with 0.5 M and 0.75 M DIEAA using an Axon Scaner;
FIG. 6D illustrates the results of scanning hybridized microarrays respectively treated with 0.5 M and 0.75 M TMAC;
FIG. 6E illustrates the results of scanning hybridized microarrays respectively treated with 1.0 M NaCl, and 5% and 25% formamide;
FIG. 7 is a graph and a table illustrating the hybridization intensity of a perfect match probe, the hybridization intensity of a mismatch probe, and a PM/MM ratio obtained from the results of FIGS. 6A through 6E;
FIG. 8 illustrates the arrangement of 20 nucleic acid probes for educational oligomer test chip when manufacturing a microarray in Example 2; and
FIGS. 9A through 9C illustrate the hybridization intensity of a perfect match probe (PM) and the hybridization intensity of a nucleic acid probe having the highest hybridization intensity among nucleic acid probes other than the perfect match probe (NM) as a bar graph, and a PM/NM ratio as a line graph for SEQ ID NO: 24 perfectly matching with SEQ ID NO: 12 (FIG. 9A), SEQ ID NO: 25 perfectly matching with SEQ ID NO: 20 (FIG. 9B), and SEQ ID No: 26 perfectly matching with SEQ ID NO: 21 (FIG. 9C), respectively.

### DETAILED DESCRIPTION OF THE INVENTION

The present invention will now be described more fully with reference to the accompanying drawings, in which exemplary embodiments of the invention are shown.

A method of increasing a ratio (PM/MM) of the hybridization intensity of a perfect match probe (PM) to the hybridization intensity of a mismatch probe (MM) while increasing or maintaining the PM according to an embodiment of the present invention includes adding a compound represented by formula (1) to a hybridization solution as a sample: 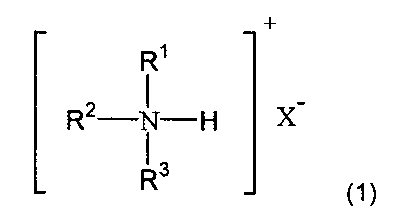 where each of R¹, R², and R³ is independently a straight or branched C₁-C₅ alkyl; and X- is an anion of an organic acid. When the number of carbon atoms in the alkyl group is greater than 5, the compound represented by formula (1) tends to solidify, and thus is not proper for application to the hybridization method.

The alkyl group may have 2 or 3 carbon atoms and examples thereof include ethyl, n-propyl, and isopropyl.

Examples of the anion of an organic acid include, but are not limited to, formate, acetate, propionate, butyrate, p-toluene sulfonate, benzene sulfonate, trifluoroacetate, oxalate, and tartrate. The compound represented by formula (1) may be, for example, triethylammonium acetate or N,N-diisopropylethylammonium acetate.

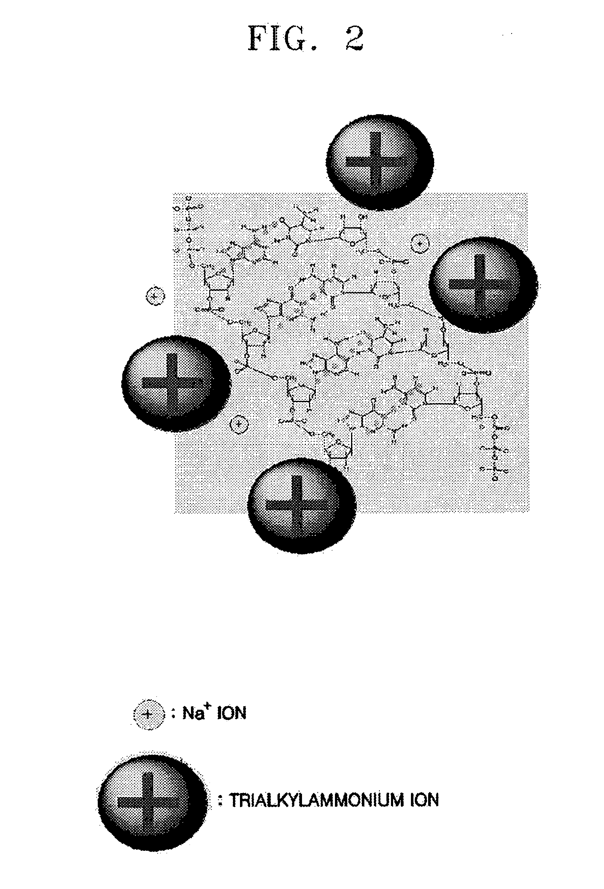

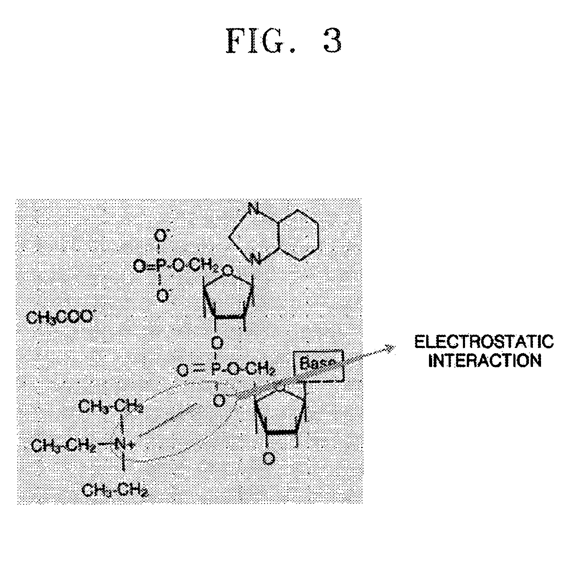

The compound represented by formula (1) may be divided into a cation of trialkylammonium and an anion of an organic acid. The compound represented by formula (1) is added to the hybridization solution to produce the trialkylammonium cation in the hybridization solution. Like a sodium cation which is typically added to a hybridization solution, the trialkylammonium cation forms an ionic bond with a phosphate anion of a hybridized gene due to an electrostatic interaction. FIG. 2 illustrates a structure in which the trialkylammonium cation derived from the compound represented by formula (1) forms an electrostatic ionic bond with an oxygen anion of phosphate of hybridized ds-DNA. FIG. 3 illustrates in detail the structure in which triethylammonium acetate, which is representative of the compound represented by formula (1), forms an electrostatic ionic bond with an oxygen anion of phosphate of hybridized ds-DNA.

The ionic bond by electrostatic interaction reduces a repulsive force between phosphates preventing hybridization of genes, thus increasing the hybridization intensity of genes.

However, if the compound represented by formula (1) acts only as a counter ion for phosphate of genes, as in the case of NaCl, it increases the hybridization intensity of a mismatch probe (MM) as well as the hybridization intensity of a perfect match probe (PM). In this case, the PM can be increased, but the PM/MM ratio cannot be increased. To use the compound represented by formula (1) as a severe condition for hybridization of genes in addition to as the counter ion for phosphate of gene, it is structurally designed such that the cation is much larger than NaCl.

That is, the compound represented by formula (1) produces a structurally large trialkylammonium cation in the hybridization solution. Such a cation is electrostatically bound to a phosphate to reduce the hybridization repulsive force caused by the phosphate, and simultaneously, an electrostatic repulsive force is produced between the trialkylammonium cations bound to the phosphate of the hybridized ds-DNA. Thus, the compound represented by formula (1) also produces a severe condition in the hybridization of genes. Such a severe condition reduces the MM more than the PM in a microarray, thereby increasing the PM/MM ratio. Furthermore, as described above, the compound represented by formula (1) does not only produce the severe condition, but also stabilizes hybridization to increase the PM. That is, the compound represented by formula (1) increases the PM/MM ratio while increasing or maintaining the PM.

When the compound represented by formula (1) is added to a hybridization solution for a gene microarray, the concentration of the compound in the hybridization solution may vary according to the specific compound represented by formula (1) used and a base sequence of a gene to be hybridized. Thus, a proper concentration of the compound represented by formula (1) may be practically determined by the optimization of experimental conditions before performing a genetic assay of a sample using a gene microarray.

An additive for a hybridization solution which is applied to a gene microarray according to an embodiment of the present invention includes the compound represented by formula (1). The additive may include only the compound represented by formula (1), and may optionally include materials commonly added to hybridization solutions. The additive can increase the PM/MM ratio while increasing or maintaining the hybridization intensity of genes in a sample to a perfect match probe in a gene chip when the hybridization solution is applied to a gene microarray, as described above in connection with the hybridization method. Thus, the additive can be used to more accurately conduct an assay for genes in the sample.

The present invention will now be described in greater detail with reference to the following examples. The following examples are for illustrative purposes only, and are not intended to limit the scope of the invention.

### Examples

### Example 1

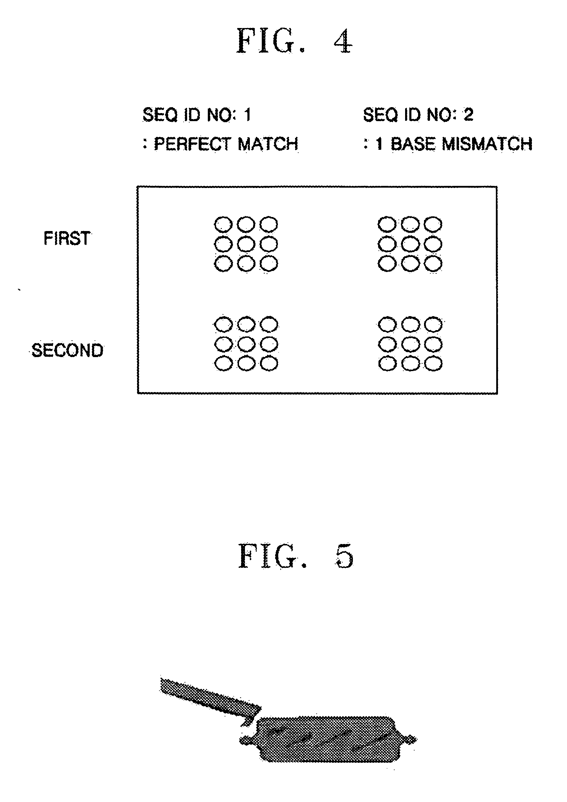

Hybridization specificity test of sample to perfect match probe and mismatch probe on gene microarray
(1) Manufacture of microarray
A nucleic acid as set forth in SEQ ID NO:1 and a nucleic acid as set forth in SEQ ID NO: 2 (100 µ mol/L, 10 µℓ) were respectively mixed with a solution containing polyethylene glycol (9 mmol/L, 10 µℓ), formamide (10 µℓ), and a buffer (0.025 mol/L, pH 10.0, carbonate 10 µℓ) and spotted (3X3) on an amine wafer. The mixtures were respectively spotted once more to achieve the arrangement illustrated in FIG. 4. The spotted substrate was kept at 70°C and a humidity of 40% for 1 hour. Then, portions of the amine wafer where the spots were not formed were deactivated using succinic anhydride, and then washed and dried to form a microarray.
(2) Preparation of hybridization solution
A. Preparation of target DNA labeled with Cy3
A nucleic acid as set forth in SEQ ID NO: 3 (target DNA) and activated Cy 3 were reacted with each other in a pH 12 solution at room temperature for 12 hours to obtain a Cy3 labeled nucleic acid. The Cy3 labeled nucleic acid was isolated using high performance liquid chromatography. The isolated nucleic acid was subjected to concentration calibration and the purity thereof was identified with a UV-vis spectrophotometer.
B. Preparation of buffer for hybridization (4X SSPET)
Sodium dihydrogen phosphate (NaH₂PO₄·H₂O), sodium chloride (NaCl), ethylene diamine tetraacetic acid (EDTA), sodium hydroxide (NaOH), and Triton X-100 were used to prepare a desired buffer, 4X SSPET (pH 7.4).
C. Preparation of additive
- Preparation of triethylammonium acetate (TEAA) solution
   Triethylamine was dissolved in water to prepare a 4 M solution, and then acetic acid was added thereto until the pH of the resultant solution was 7.3. The resultant solution was diluted by 3/4, 1/2, and 1/4 to obtain 1 M, 2 M, 3 M, and 4 M solutions.
- Preparation of N,N-diisopropylethylammonium acetate (DIEAA) solution
   Diisopropyl ethyl amine (DIEA) was dissolved in water to prepare a 3 M solution, and then acetic acid was added thereto until the pH of the resultant solution was 7.3. The resultant solution was diluted by 2/3 and 1/3 to obtain 1 M, 2 M, and 3 M solutions.
- Preparation of tetramethylammonium chloride (TMAC) solution
   TMAC available from Sigma Aldrich was dissolved in water to prepare a 3 M solution. The solution was diluted by 2/3 and 1/3 to obtain 1 M, 2 M, and 3 M solutions.
D. Preparation of hybridization solution for microarray
15 µℓ of a 4 nM Cy3 labeled target nucleic acid (such that the concentration of Cy3 labeled target nucleic acid in the final hybridization solution would be 1 nM) was mixed with 15 µℓ of triple distilled water, 15 µℓ of the buffer for hybridization (4X SSPET) (such that 1X SSPET would be in the final hybridization solution), and 15µℓ of an additive as set forth in Table 1 to obtain 60µℓ of a hybridization solution.

**Table 1**

| Experimental condition | Additive (15 µℓ) | Experimental condition | Additive (15 µℓ) |
|---|---|---|---|
| TEAA (0.25 M) | 1 M TEAA | TMAC (0.5 M) | 2 M TMAC |
| TEAA (0.5 M) | 2 M TEAA | TMAC (0.75 M) | 3 M TMAC |
| TEAA (0.75 M) | 3 M TEAA | NaCl (1.0 M) | 4X SSPET |
| TEAA (1 M) | 4 M TEAA | 5% Formamide | Formamide 3µℓ + water 12µℓ |
| DIEAA (0.5 M) | 2 M DIEAA | 25% Formamide | Formamide |
| DIEAA (0.75 M) | 3 M DIEAA | | |
| Control | Water | | |

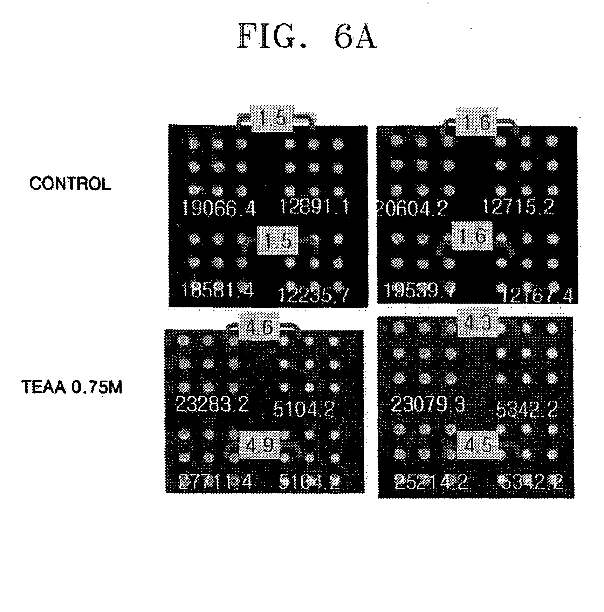

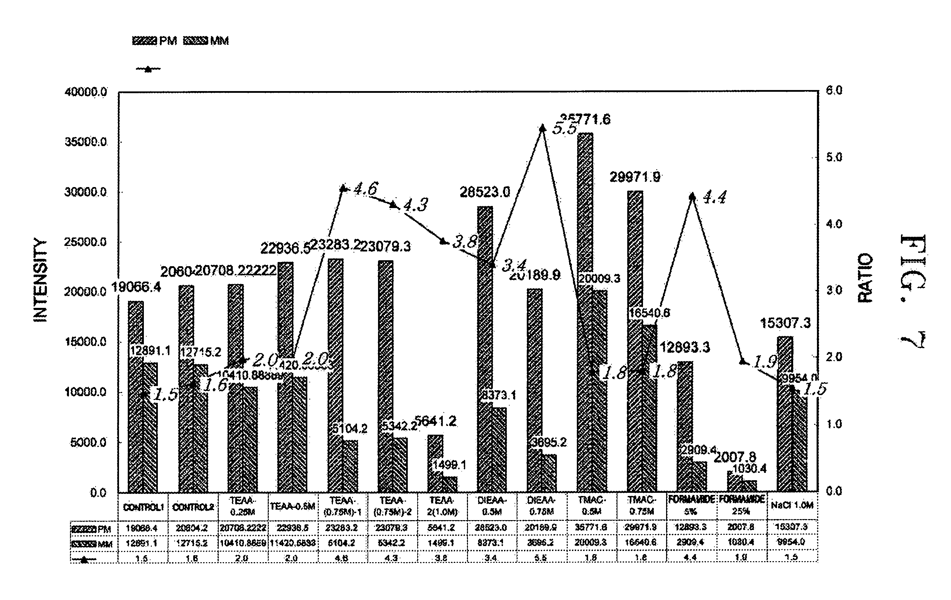

(3) Preparation of buffer for washing after hybridization (3X SSPET, 1X SSPET)
Sodium dihydrogen phosphate (NaH₂PO₄·H₂O), sodium chloride (NaCl), ethylene diamine tetraacetic acid (EDTA), sodium hydroxide (NaOH), and Triton X-100 were used to prepare buffers for washing, 3X SSPET and 1X SSPET (pH 7.4).
(4) Hybridization on gene microarray
A. Hybridization
   As illustrated in FIG. 5, a proper patch was attached to the microarray. 60 µℓ of the hybridization solution described above was introduced into the microarray through a hole at one side and bubbles were removed. The microarray was placed in a hybridization vessel for hybridization at 42°C for 1 hour.
B. Wash and dry after hybridization
   The hybridized gene microarray was washed with the buffers, 3X SSPET and 1X SSPET, for 5 minutes each. The microarray was dried using a centrifuge (300 rpm, 5 seconds).
(5) Hybridization results on gene microarray
The hybridized microarray was scanned using an Axon Scanner at a wavelength of 532 nm and PMT 500. FIGS. 6A through 6E illustrate scanning results for the respective additives.
FIG. 6A illustrates scanning results for the control and the treatment with 0.75 M TEAA. The control and 0.75 M TEAA were each tested twice and two scanning results for the respective cases are illustrated. In FIG. 6A, the hybridization intensity of a perfect match probe (PM) and the hybridization intensity of a mismatch probe (MM) obtained by analyzing the scanning results using a processing program of the Axon Scanner are indicated below each spot. The PM/MM ratio was calculated based on the PM and MM, and are shown in the boxes in FIG. 6A.

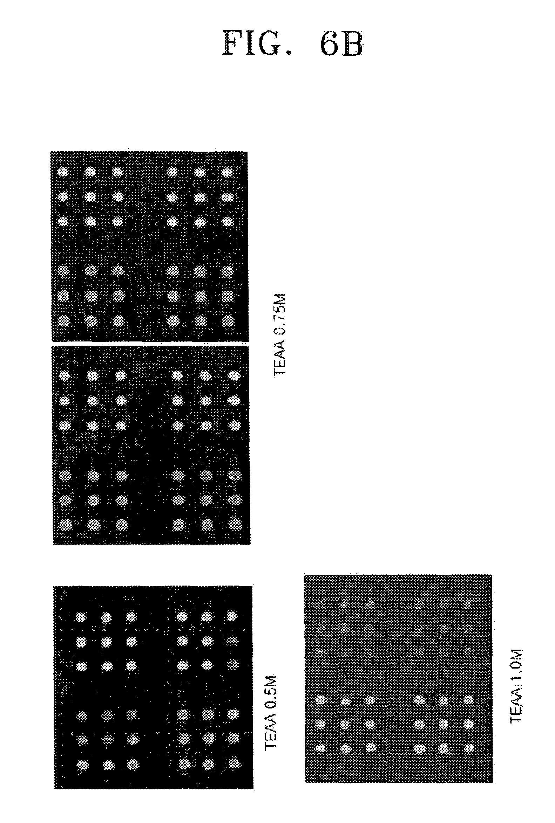

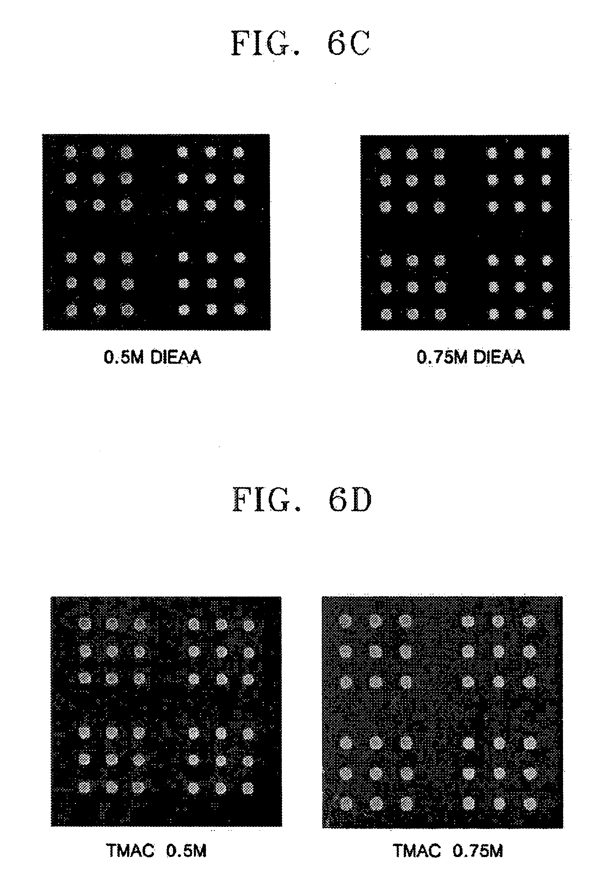

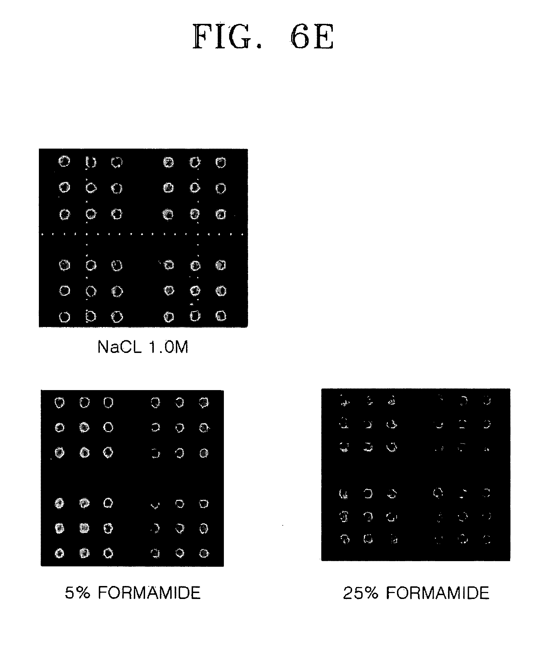

FIG. 6B illustrates the results for the treatment with 0.5 M, 0.75 M, and 1.0 M TEAA. FIG. 6C illustrates the results for treatment with 0.5 M and 0.75 M DIEAA. FIG. 6D illustrates the results for the treatment with 0.5 M and 0.75 M TMAC, which is a conventional additive. FIG. 6E illustrates the results for the addition of 1.0 M NaCl, and 5% and 25% formamide.
The scanning results of FIGS. 6A through 6E were analyzed using a processing program of an Axon Scaner to determine the respective hybridization intensities. The PM, MM, and PM/MM ratio for each additive are illustrated as a graph and a table in FIG. 7.
(6) Results
As is apparent from FIG. 7, TEAA (0.25 M, 0.5 M, and 0.75 M) and DIEAA (0.5 M and 0.75 M), which were compounds according to an embodiment of the present invention, had higher PM and PM/MM ratio than control, indicating that TEAA and DIEAA can be used as additives for hybridization specificity to a perfect match probe. In particular, 0.75 M TEAA and 0.75 M DIEAA had significantly higher PM and PM/MM ratio than control, and were thus found to be more preferable.

The conventional TMAC had higher PM, but a significantly lower PM/MM ratio than the compound represented by formula (1). Formamide, which is used to provide a conventional severe condition, had a significantly higher PM/MM ratio than the control, but very low PM, as expected. NaCl had the same PM/MM ratio as the control.

### Example 2

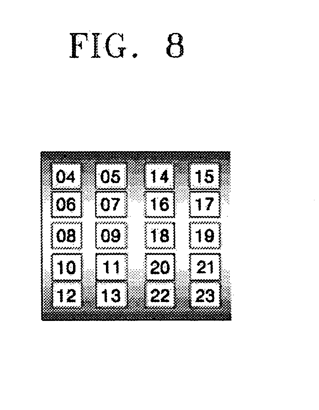

Increase in PM and specificity test in hybridization of various target samples with educational oligomer test chip having a number of probes immobilized thereon
(1) Manufacture of microarray
Nucleic acids as set forth in SEQ ID NOS: 4 to 23 (100 µ mol/L, 10 µℓ) were respectively mixed with a solution containing polyethylene glycol (9 mmol/L, 10 µℓ), formamide (10 µℓ), and a buffer (0.025 mol/L, pH 10.0, carbonate 10 µℓ) and spotted (2X3) on an amine wafer to achieve the arrangement illustrated in FIG. 8. In FIG. 8, numerals denote SEQ ID NOS. The spotted substrate was kept at 70°C and a humidity of 40% for 1 hour. Then, portions of the amine wafer where the spots were not formed were deactivated using succinic anhydride, and then washed and dried to form a microarray.
(2) Preparation of hybridization solution
A. Preparation of target DNA labeled with Cy3
A nucleic acid as set forth in SEQ ID NO: 24 (a nucleic acid having a sequence complementary to SEQ ID NO: 12), a nucleic acid as set forth in SEQ ID NO: 25 (a nucleic acid having a sequence complementary to SEQ ID NO: 20), and a nucleic acid as set forth in SEQ ID NO: 26 (a nucleic acid having a sequence complementary to SEQ ID NO: 21) were reacted with activated Cy3 in a pH 2 solution at room temperature for 12 hours to produce Cy3 labeled nucleic acids. The Cy3 labeled nucleic acids were respectively isolated using high performance liquid chromatography. The isolated nucleic acids were subjected to concentration calibration and the purity thereof was identified with a UV-vis spectrophotometer.
B. Preparation of buffer for hybridization (4X SSPET)
Sodium dihydrogen phosphate (NaH₂PO₄·H₂O), sodium chloride (NaCl), ethylene diamine tetraacetic acid (EDTA), sodium hydroxide (NaOH), and Triton X-100 were used to prepare a desired buffer, 4X SSPET (pH 7.4).
C. Preparation of additive
- Preparation of triethylammonium acetate (TEAA) solution
   Triethylamine was dissolved in water to prepare a 3 M solution, and then acetic acid was added thereto until the pH of the resultant solution was 7.3. The resultant solution was diluted by 2/3 and 1/3 to obtain 1 M, 2 M, and 3 M solutions.
D. Preparation of hybridization solution for microarray
4 nM Cy3 labeled target nucleic acids as set forth in SEQ ID NOS: 24, 25, and 26 (15 µℓ) (such that a concentration of the Cy3 labeled target nucleic acids in the final hybridization solution was 1 nM) were respectively mixed with 15 µℓ of triple distilled water, 15 µℓ of the buffer for hybridization (4X SSPET) (such that 1X SSPET was in the final hybridization solution), and 15µℓ of an additive as set forth in Table 2 to obtain 60µℓ of a hybridization solution.

**Table 2**

| Experimental condition | Type of additive (15 µℓ) |
|---|---|
| TEAA (0.25 M) | 1 M TEAA |
| TEAA (0.5 M) | 2 M TEAA |
| TEAA (0.75 M) | 3 M TEAA |

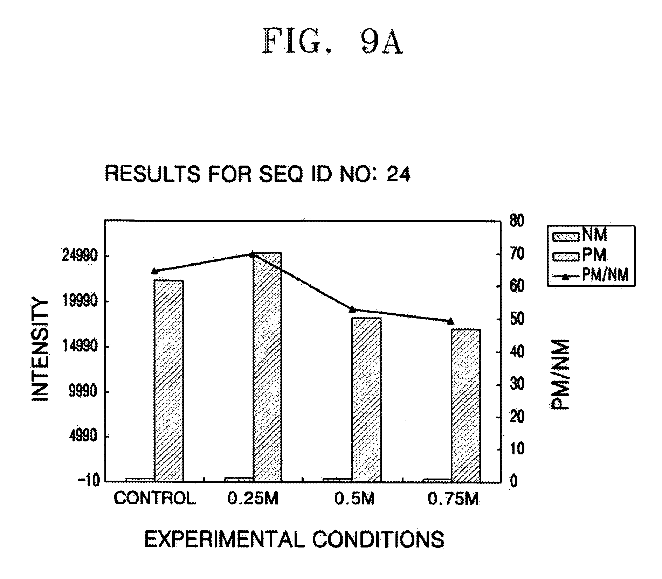

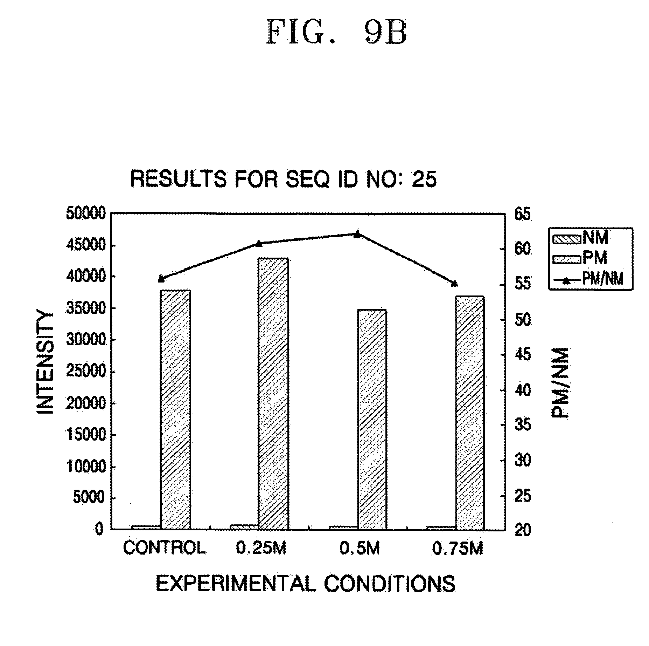

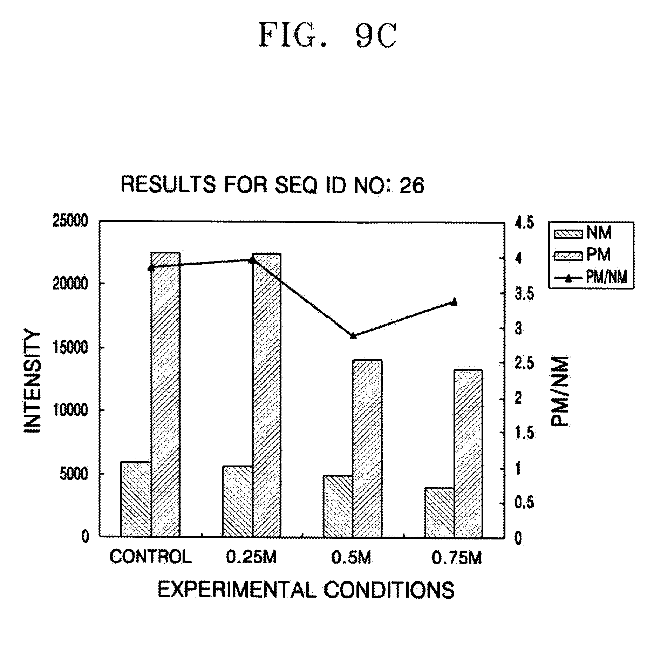

(3) Preparation of buffer for washing after hybridization (3X SSPET, 1X SSPET)
Sodium dihydrogen phosphate (NaH₂PO₄·H₂O), sodium chloride (NaCl), ethylene diamine tetraacetic acid (EDTA), sodium hydroxide (NaOH), and Triton X-100 were used to prepare buffers for washing, 3X SSPET and 1X SSPET (pH 7.4).
(4) Hybridization on gene microarray
A. Hybridization
   As illustrated in FIG. 5, a proper patch was attached to the microarray. 60 µℓ of the hybridization solution prepared above was introduced into the microarray through a hole at one side and bubbles were removed. The microarray was placed in a hybridization vessel for hybridization at 42°C for 1 hour.
B. Wash and dry after hybridization
   The hybridized gene microarray was washed with the buffers, 3X SSPET and 1X SSPET, for 5 minutes each. The microarray was dried using a centrifuge (300 rpm, 5 seconds).
(5) Hybridization results on gene microarray
The hybridized microarray was scanned using an Axon Scanner at a wavelength of 532 nm and PMT 500. The scanning results for the cases in which the TEAA additive was used in various concentrations were analyzed using a processing program of the Axon Scanner to determine the respective hybridization intensities. FIGS. 9A through 9C illustrate the PM and the hybridization intensity of a nucleic acid probe having the highest hybridization intensity among nucleic acid probes other than the perfect match probe (also called NM) when a control containing no additive and the addition of 0.25 M, 0.5 M, and 0.75 M TEAA were used as a bar graph. FIGS. 9A through 9C also illustrate line graphs of the PM/NM ratio. FIG. 9A illustrates the results for the nucleic acid as set forth in SEQ ID NO: 24 which perfectly matches with the nucleic acid as set forth in SEQ ID NO: 12. FIG. 9B illustrates the results for the nucleic acid as set forth in SEQ ID NO: 25 which perfectly matches with the nucleic acid as set forth in SEQ ID NO: 20. FIG. 9C illustrates the results for the nucleic acid as set forth in SEQ ID NO: 26 which perfectly matches with the nucleic acid as set forth in SEQ ID NO: 21.
(6) Results
As is apparent from FIGS. 9A through 9C, for all cases of nucleic acids as set forth in SEQ ID NOS: 24, 25, and 26, when TEAA was added in specific concentrations, the hybridization intensity of a perfect match probe to a target sample was high. When various target samples were used, the hybridization intensity when the compound represented by formula (1) was used as an additive in a specific concentration was higher than when the control was used, which indicates that the compound represented by formula (1) according to an embodiment of the present invention can be used to increase the PM and hybridization specificity.

As described above, the compound represented by formula (1) can be used as an additive for increasing the hybridization specificity to a perfect match probe while increasing or maintaining the PM when hybridization occurs on a gene microarray using a hybridization solution.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A method of hybridizing genes, comprising applying to a gene microarray a hybridization solution in which a compound represented by formula (1) is added: 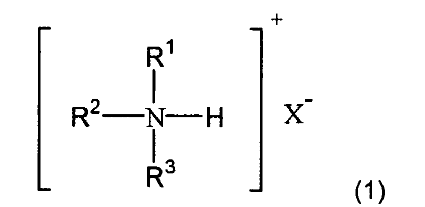 wherein each of R¹, R², and R³ is independently a straight or branched C₁-C₅ alkyl; and X- is an anion of an organic acid.

2. The method of claim 1, wherein each of R¹, R², and R³ is independently ethyl, n-propyl, or iso-propyl.

3. The method of claim 1, wherein the anion of an organic acid is formate, acetate, propionate, butyrate, p-toluene sulfonate, benzene sulfonate, trifluoroacetate, oxalate, or tartrate.

4. The method of claim 1, wherein the compound represented by formula (1) is triethylammonium acetate or N,N-diisopropylethylammonium acetate.

5. An additive comprising a compound represented by formula (1) for addition to a hybridization solution of DNA or RNA which is applied to a gene microarray: 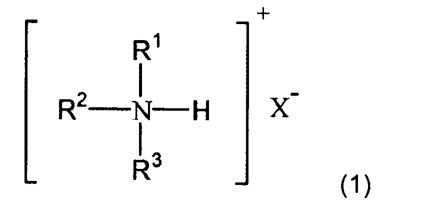 where each of R¹, R², and R³ is independently a straight or branched C₁-C₅ alkyl; and X- is an anion of an organic acid.

6. The additive of claim 5, wherein each of R¹, R², and R³ is independently ethyl, n-propyl, or iso-propyl.

7. The additive of claim 6, wherein the anion of an organic acid is formate, acetate, propionate, butyrate, p-toluene sulfonate, benzene sulfonate, trifluoroacetate, oxalate, or tartrate.

8. The additive of claim 6, wherein the compound represented by formula (1) is triethylammonium acetate or N, N-diisopropylethylammonium acetate.
